# EUROPEAN PATENT APPLICATION

(11) **EP 4 623 829 A1**
(43) Date of publication of application: **01.10.2025**
(21) Application number: 24167573.5
(22) Date of filing: 28.03.2024
(51) Int. Cl.: A61B 6/03, A61B 6/40, A61B 6/00

(54) **METHOD FOR PERFORMING KILOVOLTAGE PEAK SWITCHING COMPUTED TOMOGRAPHY, COMPUTER PROGRAM, COMPUTED TOMOGRAPHY SYSTEM, AND USE OF A RADIATION FILTER**

(71) Applicant: Koninklijke Philips N.V., 5656 AG Eindhoven (NL)
(72) Inventor: PROKSA, Roland, Eindhoven (NL)
(74) Representative: Philips Intellectual Property & Standards

(57) **Abstract**

A method for performing kilovoltage peak switching computed tomography with a computed tomography system comprising an x-ray source (2), the method comprising the following steps:
(a) receiving user input for a kilovoltage peak switching computed tomography scan, the user input comprising a desired radiation dose setting and a desired speed setting of the scan;
(b) setting computed tomography scan parameters based on the received user input;
(c) automatically determining whether a radiation filter (6) is required to achieve the desired radiation dose setting and desired speed setting and, if required, placing the radiation filter (6) in front of the x-ray source (2) to achieve the desired radiation dose with the set scan parameters;
(d) performing a kilovoltage peak switching computed tomography scan with the set scan parameters.

## Description

### FIELD OF THE INVENTION

The invention relates to a method for performing kilovoltage peak switching computed tomography and a corresponding computer program, computed tomography system and use of a radiation filter.

### BACKGROUND OF THE INVENTION

Spectral computed tomography (CT) imaging is a variant of computed tomography imaging that uses different x-ray spectra to differentiate between different materials. This technique makes use of the fact that the x-ray attenuation tends to have a different energy- dependance for different materials. When using two different spectra, spectral computed tomography is also referred to as dual-energy computed tomography.

A relatively simple and cheap approach for spectral CT imaging is rapid kVp-switching (kVp-S; "kVp" stands for kilovoltage peak), also denoted as fast kVp switching. The kilovoltage peak refers to the maximum voltage that is applied in an x-ray source of a CT system in order to generate the x-rays. The voltage is also referred to as tube voltage or tube acceleration voltage. For generating the x-rays in CT, typically, electrons are emitted from a heated cathode and accelerated by the applied voltage (typically in the range of kilovolt, hence kilovoltage peak) towards an anode. The electron current of the electron beam corresponds to an emission current, also denoted as tube current, tube emission current, or anode current. While the electrons are stopped in the anode, x-rays are produced with an x-ray spectrum that has a maximum photon energy being proportional to the applied kilovoltage peak and that further depends on the anode material. Fast kVp-S CT is based on using a single x-ray source and quickly alternating, i.e. switching, the tube acceleration voltage between two distinct kVp levels.

However, kVp-S is challenging for fast low-dose imaging. Fast imaging requires short switching cycles to acquire enough projections during a short scanner rotation time. To realize low-dose imaging, the tube emission current is set to be small, since the radiation dose directly depends on the tube emission current. A larger emission current generally leads to a larger amount of radiation. Small emission currents will generate long voltage transition times from high to low tube acceleration voltages. This is because the falling transition time is mainly defined by the output capacitance of the generator and the emission current that discharges this capacitor. During the transition times, the voltage is in between the first kilovoltage peak and the second kilovoltage peak. Increased rise and fall times of the voltage may decrease the spectral separation. A good material differentiation typically requires a strong difference between the photon energies of the applied x-ray spectra and, thus, of the applied voltages, which corresponds to a strong spectral separation. For fast switching and low dose applications (i.e., requiring a low emission current and thus leading to a slower lowering of the voltage) the transition times may become so long that the spectral performance, that depends on a good spectral separation between the kilovoltage peaks, may be impaired significantly. Furthermore, in particular for low-dose applications, even a lower limit may be reached where kVp-switching cannot be performed any more with a desired speed.

### OBJECT OF THE INVENTION

It is, therefore, an object of the invention to provide a method to better address the above-mentioned problems. In particular, it would be desirable to find a solution that enables faster kVp switching while still allowing low-dose imaging.

### SUMMARY OF THE INVENTION

To better address this object, a method according to claim 1, a computer program according to claim 11, a computed tomography system according to claim 12 and a use of a radiation filter according to claim 15 is provided. Advantageous embodiments are set out in the dependent claims. Any features, advantages or alternative embodiments described herein in relation to the claimed method are also applicable to the other claim categories and vice versa.

According to a first aspect, a method for performing kilovoltage peak switching computed tomography (kVp-S CT) with a computed tomography (CT) system comprising an x-ray source is provided. The method comprises the following steps:
(a) receiving user input for a kilovoltage peak switching computed tomography scan, the user input comprising a desired radiation dose setting and a desired speed setting of the scan;
(b) setting computed tomography scan parameters based on the received user input;
(c) automatically determining whether a radiation filter is required to achieve the desired radiation dose setting and desired speed setting and, if required, placing the radiation filter in front of the x-ray source to achieve the desired radiation dose with the set scan parameters;
(d) performing a kilovoltage peak switching computed tomography scan with the set scan parameters.

In particular, the method steps may be performed in the given order.

Advantageously, with the method according to the invention, a fast kVp-S CT scan may be performed for low dose applications, even in some cases where the transition time of the tube voltage would otherwise become too long to reach the timing and dose constraints for fast low-dose imaging. This is in particular achieved by flexibly choosing and placing the radiation filter. Hence the radiation filter may be chosen and/or changed depending on the set scan protocol. In the context of this invention, the "radiation filter" may also be denoted as simply "filter".

The CT system may have components according to known CT systems. In particular the CT system may comprise a gantry with the x-ray source and an x-ray detector. The x-ray detector may also be referred to as "detector" within the context of this invention. The x-ray source may also be referred to as "source" within the context of this invention. The gantry may be a CT gantry as generally known in the state of the art. A gantry may in particular be a short ring tunnel that is configured such that a subject or an object (e.g., a patient or part of a patient) can be placed inside the area encompassed by the gantry.

The x-ray spectra used for the kVp-S CT are generated by the x-ray source. The x-ray source may be any x-ray source suitable for kVp-S CT. In particular an x-ray source as known from the state of the art may be used. The x-ray source may comprise a cathode and an anode to generate the x-rays. The x-ray source is in particular configured to rapidly switch the tube voltage. Preferably, the x-ray source is configured to provide a substantially steady kilovoltage peak in between voltage switches, providing voltage plateaus of the kVp-S. For the purpose of spectral CT kVp-S may be performed by providing a switching of voltage between multiple kVp plateaus, in particular between two plateaus. For example, a first kilovoltage peak may be in the range of 110 kVp to 1000 kVp, preferably 120 kVp to 200 kVp, more preferably 130 kVp to 150 kVp. For example, a second kilovoltage peak may be in the range of 30 kVp to 100 kVp, preferably 50 kVp to 95 kVp, more preferably 60 kVp to 90 kVp. For example, an upper and lower kVp may be set depending on the material that is to be scanned, in particular depending on the attenuation properties of the material.

The method comprises a step of receiving user input for a kilovoltage peak switching computed tomography scan. The user input may, for example, be received via a user interface that is configured to enable such input or from another user input functionality. The user input comprises a desired radiation dose setting and a desired speed setting of the scan. The radiation dose may, for example, depend on the object or subject to be examined. The subject, may, for example be an animal or human being, in particular a patient. An object may, for example, be a lifeless item or part of an animal or human being. Generally, limiting radiation dose is particularly important for life subjects, such as animals or human beings. However, in some cases limiting the radiation dose may also be relevant for lifeless items, e.g. in order to prevent damage to sensitive objects. For example, a thickness of the object or subject may be used to determine the radiation dose. For example, larger body parts and/or obese patients may require a higher radiation dose, such that a significant enough part of the radiation can still penetrate the patient and be registered by the detector. On the other hand, for example, children, small body parts and/or thin patients may require a lower radiation dose. The desired radiation dose may depend on patient safety regulations. The speed setting may depend on an expected movement of the examined subject or object.

Depending on the user input computed tomography scan parameters are automatically set. The scan parameters may be set by a control processing circuitry of the computed tomography system. The scan parameters may comprise typical scan parameters as known from the state of the art. An algorithm used for setting the scan parameters may be based on a known algorithm that is known from the state of the art. For example, the scan parameters may comprise one or a combination of the following: a detector configuration, kVp-S settings, such as multiple kVp values and/or switching speed, an emission current, a patient positioning, a scan range, a chosen reconstruction algorithm, a slice thickness etc. In particular a relatively high emission current may be set. This high emission current may be enabled due to the possibility to flexibly provide a filtering via the radiation filter and thus adjust the dose application that is applied on the object or subject. The scan parameters may define a scan protocol or part of a scan protocol that is to be carried out. Setting the scan parameters may take into account the possibility of determining and placing a radiation filter. Hence, the scan parameters may be set such that they are intended to work together with a corresponding radiation filter. The scan parameters may comprise an indication of a radiation filter to be used. The radiation filter may be a radiation filter from a set of available radiation filters.

Based on the set scan parameters and the desired radiation dose setting, it is automatically determined whether a radiation filter is required to achieve the desired radiation dose setting. The determination may be performed by the same control processing circuitry of the computed tomography system that sets the scan parameters. Alternatively, the determination of the radiation filter may be performed by a further processing circuitry. For example, if setting the scan parameters comprises indicating a radiation filter to be used, the determination of the radiation filter may comprise checking for the indication and, if there is an indication to use a radiation filter, determine that the indicated radiation filter is to be used. Optionally, the determination may be performed at the same time as setting the scan parameters. For example, an iterative process may be applied that determines which scan parameters work best with which available radiation parameter. Hence, these two steps of setting parameters and determining, whether a radiation filter is required are to be understood broadly. The computed tomography system (e.g. in the form of a control processing circuitry) may thus be configured to decide which scan mode and with or without filter is estimated to be optimal for the user input. Advantageously, the computed tomography system may thus apply and/or change the radiation filter depending on a scan protocol that is to be performed.

The radiation filter is designed to filter x-rays in the range of the x-ray spectra generated by the x-ray source. The radiation filter may comprise a single filter material or may comprise a plurality of filter materials.

If it is determined that a radiation filter or a specific radiation filter out of multiple available radiation filter is required and/or is beneficial, the radiation filter is placed between the x-ray source and the subject to be imaged to lower the radiation dose with the set scan parameters. Consecutively, a kilovoltage peak switching computed tomography scan with the set scan parameters is performed. Advantageously, with the radiation filter, a low dose applied on the object or subject to be examined can be realized while still a relatively high emission current can be applied. This may enable a faster switching of the kVp, in particular a faster switching from high kVp values to low kVp values. The x-ray source may thus operate at a relatively high dose, while the actually applied dose is lower. Since the spectral performance typically depends on a distinction between different x-ray spectra, i.e. the energy spectra generated by the different kVp values, fast transition times, that allow a better separation and depend on the emission current, may thus be achieved.

According to an embodiment, the radiation filter is adapted to improve a spectral imaging performance. The image radiation filter may be chosen such that the spectral imaging performance can be enhanced. For example, the radiation filter may be configured such that the spectral separation between the high kVp and the low kVp is improved. In particular, filter materials with a suitable k-edge may shift the x-ray spectrum to lower energies and/or prevent beam hardening. Beam hardening may shift the spectrum towards higher energies. Some filter materials, such as rare earth materials like Erbium or Holmium can have k-edge filtration effects in a relevant spectral energy range. This effect can shift the x-ray spectrum towards lower energies and/or limit a hardening effect. This may be used to an advantage, e.g. when considering that higher kVp values tend to generate a larger x-ray dose than lower kVp values. Hence, typically the lower kVp tends to generate a weaker output. For example, a typical output of the lower kVp may be about 1/7 of the output of the higher kVp. However, for optimal material decomposition, both spectra, i.e. the low kVp spectrum and the high kVp spectrum are equally needed. Thus, the low kVp beam tends to be more problematic for material decomposition. Hence, for example, a radiation filter may be used that primarily filters higher x-ray energies, in particular x-ray energies that are above a level corresponding to the lower kVp. By filtering high energies, the x-ray spectrum of the low kVp may be less affected by the filter or even be cleaner due to the filtering, while the x-ray spectrum of the high kVp may have enough energy to still be useful enough even if some of the high energy photons are filtered out.

According to an embodiment, a performance relation between a spectral imaging performance and a radiation dose, the relation depending on a tube emission current of the x-ray source and radiation filter setting, is retrieved and/or determined, wherein determining whether a radiation filter is required is further based on the performance relation. The radiation dose may be normalized with respect to a maximum x-ray tube power of the system. The performance relation may be based on a simulated calculation. Optionally, the simulation may take into account the object or subject to be examined, in particular a material and/or geometry of the object or subject to be examined. For example, the simulation calculation may search for a maximum spectral performance, in particular a maximum SNR. The tube emission current may be a free parameter that is chosen to maximize the spectral imaging performance. Similarly, a radiation filter, e.g. a radiation filter out of an available set of radiation filters, may be chosen to maximize the spectral imaging performance. For example, the performance relation may be a function of the spectral imaging performance over the radiation dose. The calculated radiation dose may be determined based on the emission current and the radiation filter. The performance relation may comprise multiple functions, in particular one function for each available radiation filter. For example, the functions for different filters may be compared at a particular radiation dose, and the filter may be chosen that provides the best spectral imaging performance at this radiation dose as determined by the functions. Via the performance relation it may thus be possible to determine a maximum spectral performance for a given allowable radiation dose and available radiation filters relatively easily. The radiation dose may, for example be a singular value or a value range. The radiation dose may in particular be as defined by the user input.

According to an embodiment, the performance relation further depends on a duty cycle for time periods of the low and high tube voltage intervals of the kilovoltage peak switching. The duty cycle may define the temporal course of the tube voltage. Advantageously, by taking the duty cycle into account, a more precise estimation of the spectral imaging performance may be possible. The duty cycle may be adapted to change the dose level for a given scan speed. For example, the duty cycle may be adapted as one of the scan parameters. For example, in order to achieve a higher radiation dose, the duration of the high kVp may be increased at the cost of the duration of the low kVp. On the other hand, in order to achieve a lower radiation dose, the duration of the low kVp may be increased at the cost of duration the high kVp. This may enable a finetuning of the radiation dose to enable a particular desired radiation dose at a given scan speed. Typically, the changing of the duty cycle may be detrimental to the spectral performance. Hence, having a duration of the high kVp that is too short or a duration of the low kVp that is too long may decrease the spectral performance. Advantageously, the combination of determining a radiation filter, in particular choosing from a set of radiation filters, may allow to avoid greater changes to the duty cycle, and enable greater differences in the radiation dose via choosing the filter. Hence, for example, this embodiment may combine the advantages of improved performance by flexibly choosing a filter for greater changes to the radiation dose and the advantage of being able to finetune the radiation dose via the duty cycle.

According to an embodiment, the performance relation is based on a signal to noise ratio in relation to radiation dose. The spectral performance may be defined via the signal to noise ratio (SNR). The signal to noise ratio may, for example be normalized with respect to a reference performance and/or reference signal to noise ratio. Advantageously, a SNR may provide an efficient means of defining and classifying the spectral performance. The Signal to noise ratio may be calculated based on a simulation of the system.

According to an embodiment, a minimum tube emission current of the x-ray source that is required for kilovoltage peak switching computed tomography at the desired speed setting of the scan is retrieved and/or determined, wherein it is determined that the radiation filter is required if the desired radiation dose setting cannot be achieved above the minimum tube emission current without the filter. For example, the minimum tube emission current may be determined based on the performance relation or based on other means. Advantageously, with this embodiment, a lower dose at a particular high scan speed may be achievable via using the radiation filter. Optionally, it may be feasible to only determine whether the filter is needed to generally enable a particular combination of scan speed and radiation dose.

According to an embodiment, it is determined that the radiation filter is required if placing the radiation filter increases the expected spectral imaging performance for the desired radiation dose setting. According to this embodiment, the step of determining whether the radiation filter is required can also be used to improve the spectral imaging performance. For example, the performance relation may be used to determine the expected spectral imaging performance. However, other means may also be feasible. For example, a database of scan speeds and radiation dose with one or multiple assigned radiation filters may be used. For each set of scan speed value and radiation dose a particular optimal filter, or in some cases no filter, may be assigned. For example, each available filter may be associated with a range of scan speeds and radiation doses, where applying this filter is advantageous.

According to an embodiment, the radiation filter is chosen from a plurality of radiation filters. For example, there may be a set of different radiation filters the radiation filter is chosen from. The radiation filters may in particular differ in terms of their filter properties. The filter properties may comprise a filtered energy range and/or a filter transmittance. For example, the different radiation filters may differ in terms of their material. For example, the different radiation filters may differ in terms of their thickness. There may be the option to choose a combined filter out of the plurality of radiation filters. A combined filter is in particular a radiation filter that consists of two or more radiation filters placed one after the other to achieve a combined filter effect of both filters. Hence, a combined filter may be chosen that is a combination of multiple radiation filters from the plurality of radiation filters.

According to an embodiment, in case that a radiation filter is determined to be required, a radiation filter out of a plurality of radiation filters is chosen that provides the maximum spectral imaging performance of the plurality of radiation filters for the desired radiation dose setting. The maximum spectral imaging performance may, for example be determined based on the performance relation or based on other means.

According to an embodiment, the radiation filter has a K-edge of at least 20 keV, preferably at least 35 keV, more preferably at least 45 keV, and even more preferably at least 55 keV. Preferably, the K-edge should not be too high. For example, the K-edge may preferably be chosen to be below 100 keV, more preferably, below 90 keV. When x-ray photons have an energy corresponding to the binding energy of K-shell electrons of the atoms of a material, i.e. the electrons with the principal quantum number n= 1, photoelectric absorption based on these K-shell electrons can occur. For this reason, x-ray photons with an energy are just above the binding energy of the K-shell electrons are therefore more likely to be absorbed than photons with an energy just below the binding energy of the K-shell electrons. In x-ray absorption spectroscopy, this appears as a sudden increase of the x-ray absorption at the energy of the K-shell electrons, namely at the K-edge. The energy of the K-edge depends on material of the filter and can be adjusted by choosing a suitable material for the filter. By using a filter with a higher K-edge, the filter effect of the filter for photons with a higher energy is greater. This can be advantageous to achieve a better spectral imaging performance. Typically, applying the higher kilovoltage peak leads to a higher radiation dose than applying the lower kilovoltage peak. Hence, filtering more of the higher energy photons and fewer of the lower energy photons can be advantageous in order to not weaken the lower energy spectrum that tends to be weaker any way. Furthermore, by filtering higher energies, the energy separation between the two applied spectra may even improve, because while the lower energy spectrum is typically shifted to lower energies, the higher energy spectrum may still have a sufficient amount of higher energy x-ray photons. Thus, the spectral imaging performance may be further improved.

According to an embodiment, the radiation filter comprises a material with an atomic number of at least 50, preferably at least 60. An atomic number of around 50 may correspond to a K-edge of about 30keV. An atomic number of around 60 may correspond to a K-edge of around 44 keV. Hence, a filter material with at least an atomic number according to this embodiment may be suitable to achieve a suitable filtering effect and/or improve spectral imaging performance.

According to an embodiment, the radiation filter comprises a rare earth material and/or tungsten. For example, the rare earth material may be chosen to be Erbium, Holmium or Gadolinium. These materials have a relatively high atomic number z that leads to a relatively high K-edge absorption. Hence, these materials may be suitable to both provide an appropriate filtering effect as well as improve a spectral imaging performance.

According to another aspect of the invention, a computer program comprising instructions which, when the program is executed by a control circuitry of a computed tomography system, cause the computed tomography system to carry out the method as described herein. The instructions may comprise any of the method steps and their variants as described herein. The computer program may be stored on a data storage medium, in particular a non-transient data storage medium.

According to another aspect of the invention, a computer-readable data storage medium, in particular non-transient data storage medium, comprising instructions which, when executed by a control circuitry of a computed tomography system, cause the computed tomography system to carry out the method as described herein. The data storage medium may comprise the computer program as described herein. The data storage medium may be any computer-readable storage medium. For example, the data storage medium may be a solid-state storage medium or an optical storage medium. The storage medium may be a hard-drive, a solid-state-disk, a compact disk, a DVD, a flash storage an online server, a cloud, etc.

According to another aspect of the invention, a computed tomography system is provided. The system comprises
- a gantry, in particular rotatable gantry, with an x-ray source;
- a control circuitry configured to control a computed tomography scan performed by the computed tomography system;
- a user input functionality, optionally comprising a user interface, for obtaining user input to choose settings concerning a kilovoltage peak switching computed tomography scan comprising a desired radiation dose setting and a desired speed setting of the scan, wherein the control circuitry is configured to automatically set scan parameters depending on the user input; the user input comprises in particular the settings chosen by the user;
- at least one radiation filter, wherein the computed tomography system is configured to automatically determine whether a radiation filter is required to achieve the desired radiation dose setting and, if required, place the at least one radiation filter in front of the x-ray source depending on the set scan parameters and the desired radiation dose setting;
wherein the computed tomography system is optionally configured to perform the method as described herein.

The system may comprise the computer program and/or the computer-readable data storage medium as described herein. For example, the control circuitry may be part of a control station of the system. The control station may be a local control station or a remote control station. The control station does not have to be a unitary control station, but parts of it may optionally be spread over different locations. The user input functionality may be configured to obtain the user input from a built-in user interface or from another device which is not part of the claimed system. For example, the user input functionality and/or the user interface may comprise a user menu with corresponding options to choose or to set. If the system comprises exactly one radiation filter, the system may be configured to determine, whether the radiation filter is needed to achieve the set user input and/or whether the radiation filter is advantageous to improve spectral imaging performance based on the user input. Alternatively, the system may comprise multiple radiation filters. The radiation filter or the multiple radiation filters may be adapted to improve a spectral imaging performance, in particular for the set radiation dose setting.

According to an embodiment, the computed tomography system comprises a set of radiation filters and is configured to, if a radiation filter is determined to be required, chose a radiation filter of the set of radiation filters according to a defined filter suitability criterion. The suitability criterion may, for example, concern one or several of the following: spectral imaging performance, realization of a particular scan speed in combination with realization of a particular radiation dose (e.g., maximum radiation dose or range of radiation dose or specific number of radiation dose). The suitability criterion may in particular comprise choosing a radiation filter that provides a maximum spectral imaging performance of the plurality of radiation filters for a set radiation dose setting. The set of radiation filters may comprise radiation filters that comprise different materials. The properties of the radiation filters may, for example, differ due to different atomic numbers of the materials and/or due to different K-edges of the radiation filters and/or due to different thicknesses of the radiation filters.

According to an embodiment the radiation filter or the radiation filters of the set of radiation filters has/have a K-edge of at least 20 keV, preferably at least 35 keV, more preferably at least 45 keV, even more preferably at least 55 keV. For example, the radiation filter or the set of radiation filters may each comprise tungsten and/or a rare earth material, such as Erbium, Holmium and/or Gadolinium.

According to another aspect of the invention, a use of a radiation filter for lowering the radiation dose during a kilovoltage peak switching computed tomography (kVp-S CT) scan depending on a desired radiation dose setting and a desired scan speed setting is provided. The use may comprise using a set of radiation filters. The use may comprise automatically choosing one radiation filter of the set of radiation filters that is expected to enable the best spectral imaging performance of the kVp-S CT scan. The use may be applied as described within, in particular as described with respect to the method and/or other aspects of the invention.

The features and advantages of different embodiments can be combined. The features and advantages of one aspect of the invention, e.g., the method, may be applied to the other aspects, e.g., the computer program the storage medium, and the computed tomography system, and vice versa.

### BRIEF DESCRIPTION OF THE DRAWINGS

The invention shall now be illustrated by means of embodiments with reference to the attached drawings, in which:
Fig. 1 shows a flow diagram of a method for performing kilovoltage peak switching computed tomography according to an embodiment of the invention;
Fig. 2 shows an exemplary simplified excerpt of the course of the tube voltage of a kilovoltage peak switching computed tomography scan;
Fig. 3 shows a performance relation between a spectral imaging performance and a radiation dose according to an embodiment of the invention;
Fig. 4 shows an exemplary simplified excerpt of the course of the applied tube voltage of a kilovoltage peak switching computed tomography scan according to another duty cycle than the one shown in Fig. 2;
Fig. 5 shows an exemplary simplified excerpt of the course of the applied tube voltage of a kilovoltage peak switching computed tomography scan according to another duty cycle than the ones shown in Fig. 2 of Fig. 4;
Fig. 6 shows an exemplary simplified excerpt of the course of the applied tube voltage of a kilovoltage peak switching computed tomography scan, wherein the emission current is reduced compared to the example in Fig. 2;
Fig. 7 shows a schematic sectional view from the side of a computed tomography system according to an embodiment of the invention.

### DESCRIPTION OF EMBODIMENTS

Throughout the Figures, the same or corresponding features/elements of the various embodiments are designated with the same reference numbers.

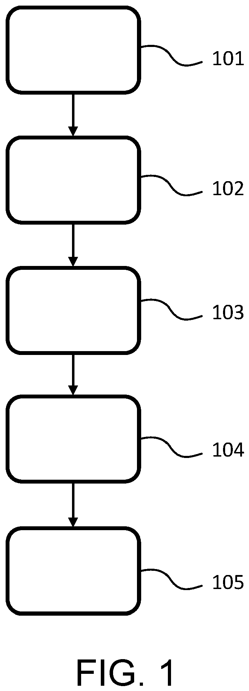

Fig. 1 shows a flow diagram of a method for performing kilovoltage peak switching computed tomography according to an embodiment of the invention. In a first step 101, user input for a kilovoltage peak switching computed tomography scan is obtained. The user input comprises a desired radiation dose setting and a desired speed setting of the scan. For example, the user input may be obtained by a user input functionality, for example, a user interface 5. The user interface 5 may comprise a menu that enables the user to choose and/or set the radiation dose setting and the speed setting. In a further step 102, depending on the user input, computed tomography scan parameters are set automatically. The setting of the scan parameters may be generally performed analogously as is already known in the state of the art. In a further step 103, depending on the set scan parameters and the desired radiation dose setting, it is automatically determined, whether a radiation filter 6 is required to achieve the desired radiation dose setting. The radiation filter 6 may be adapted to improve a spectral imaging performance, in particular by improving the spectral separation between the x-ray spectra of the higher and the lower kilovoltage peak of the kilovoltage peak switching. Optionally, it may also be determined that the radiation filter 6 is required, if placing the filter 6 increases the expected spectral imaging performance for the desired radiation dose setting. Optionally, a radiation filter 6 out of a plurality of radiation filters 6 may be chosen that is most suitable to enable the radiation dose at the desired speed and/or that provides the maximum spectral imaging performance of the plurality of radiation filters 6 for the desired radiation dose setting. In a further step 104, if it is determined that a radiation filter 6 is required and/or as soon as the most suitable radiation filter 6 is chosen, the corresponding radiation filter 6 is placed in front of the x-ray source 2 to lower the radiation dose with the set scan parameters. The radiation filter 6 may, for example be placed in front of the x-ray source 2 automatically. For example, the system may comprise a mechanism that picks and sets the filter 6 automatically. In a further step, a kilovoltage peak switching computed tomography scan is performed with the set scan parameters and the placed filter 6.

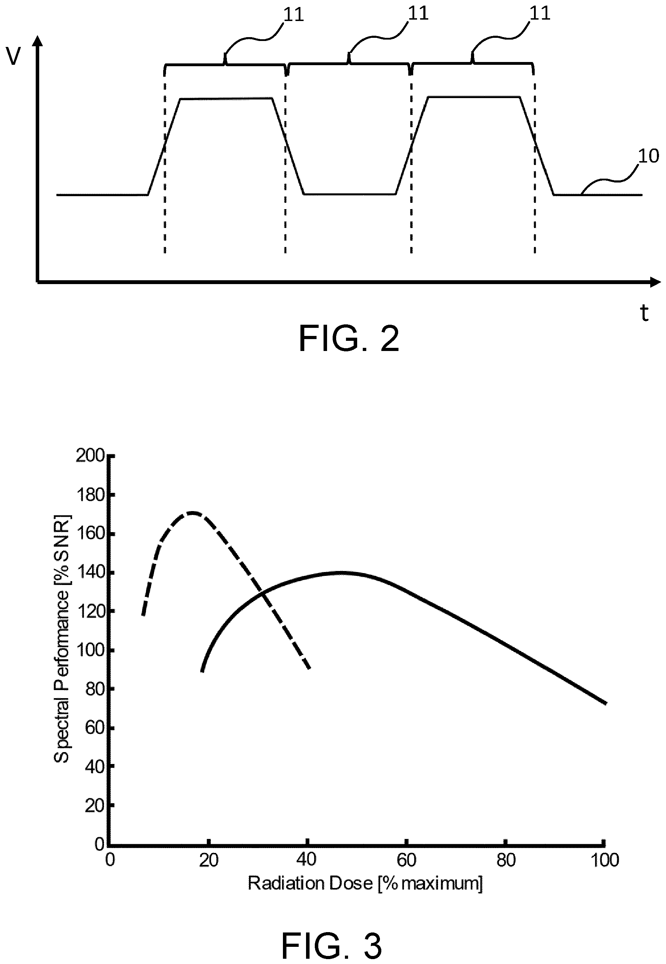

Fig. 2 shows the course of the applied tube voltage of the kilovoltage peak switching. The vertical axis corresponds to the applied tube voltage 10 and the horizontal axis corresponds to the time. Dashed lines indicate the measurement intervals 11 of the x-ray detector 3 over which the x-ray detector 3 integrates. As can be seen, the tube voltage 10 switches between a higher voltage plateau, corresponding to the higher kilovoltage peak, and a lower voltage plateau, corresponding to the lower kilovoltage peak. In between the voltage plateaus, there are transition phases, wherein the voltage transitions from one plateau to the other. The overall switching rate typically corresponds to the scan speed and the length of the measurement intervals. The applied tube voltage 10 is used to accelerate electrons towards an anode of the x-ray source 2 and determines the speed of the electrons when hitting the anode. Via the interaction of the electrons with the anode, x-ray spectra are generated. The x-ray spectra depend on the energy of the electrons and thus on the applied voltage 10, wherein the maximum energy of each x-ray spectrum corresponds to the currently applied voltage 10. The spectral imaging performance depends on the spectral separation between the two spectra (i.e. corresponding to the two voltage plateaus) and may also depend on the acquired intensity of each plateau during the measurement intervals 11.

Fig. 3 shows a performance relation between a spectral imaging performance and a radiation dose according to an embodiment of the invention. Shown are both, a spectral imaging performance without a filter 6 (solid line) and a spectral imaging performance with a radiation filter 6 placed in front of the x-ray source 2 of the computed tomography system (dashed line). The performance relation is based on a signal to noise ratio (SNR) on the vertical axis in relation to radiation dose on the horizontal axis. The Spectral imaging performance is given in per cent of the SNR relative to a detector-based spectral CT scanner using the same dose. The radiation dose is specified in % of the maximum x-Ray tube power of the CT system. The plotted SNR is an estimated performance constrained by a particular example system and shows realistic performance SNR values. In this embodiment, the performance reaches the maximum at about 45% does. If the requested radiation dose is higher than this optimum, the protocol may, for example, change the duty cycle of kVp-S towards longer high voltage periods to reach the requested dose level.

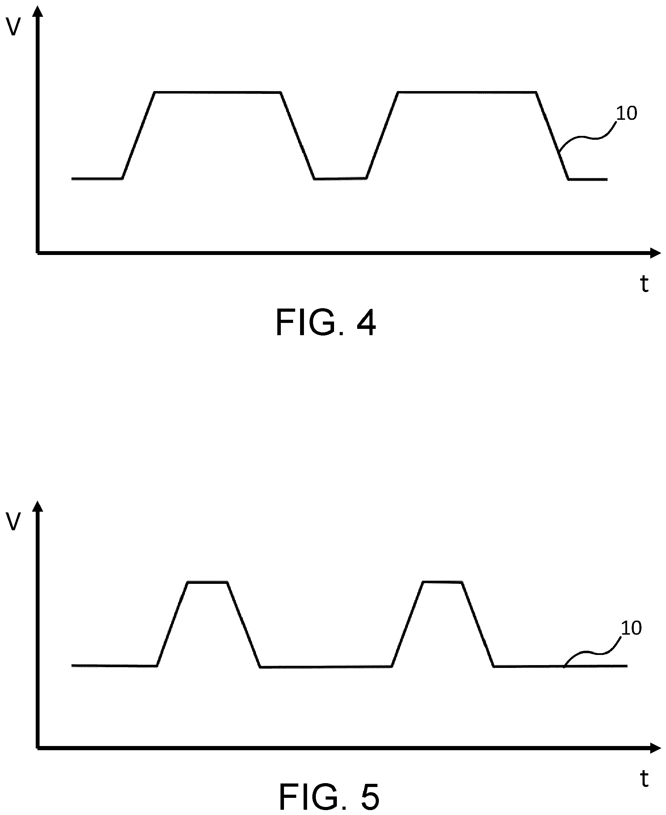

An example for this changed duty cycle is shown in Fig. 4, where, compared to Fig. 2, the duty cycle is changed such that the high voltage periods are longer. In particular, the high voltage periods are longer than the low voltage periods. Since applying higher voltages increases the dose of the generated x-ray radiation, the radiation dose may thus be increased, without changing the overall switching rate of the kVp switching. Hence, the scan speed may remain about the same.

Referring to Fig. 3 again, it can be seen that such an increase reduces the performance, i.e. the SNR decreases for radiation dose values higher than about 45%. This decrease in SNR is due to the imbalance of the acquisition times corresponding high voltage plateaus (which are longer in the duty cycle according to Fig. 4) and low voltage plateaus (which are shorter according to the duty cycle according to Fig. 4). The imbalance of the acquisition times reduces more and more the SNR. On the other hand, if the requested radiation dose is below the optimum value of about 45%, the performance also decreases. The achieve lower radiation dose, the duty cycle of kVp-S may be changed towards longer periods for the lower high voltage periods.

An example for this changed duty cycle is shown in Fig. 5, where, compared to Fig. 2, the duty cycle is changed such that the low voltage periods are longer. In particular, the low voltage periods are longer than the high voltage periods. Since applying lower voltages decreases the dose of the generated x-ray radiation, the radiation dose may thus be decreased, without changing the overall switching rate of the kVp switching. Hence, the scan speed may remain about the same.

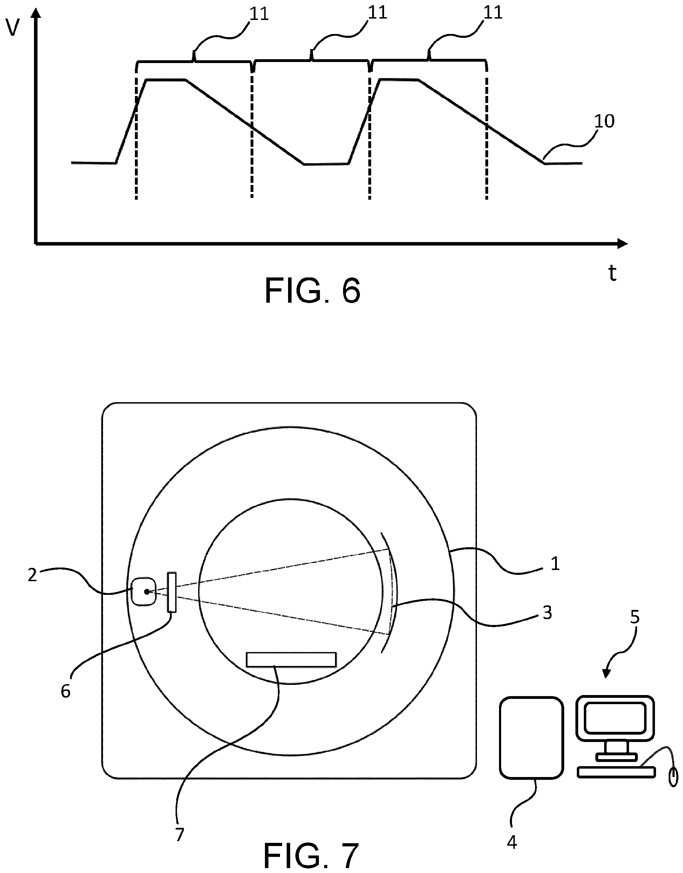

Referring to Fig. 3 again, it can be seen that the reduction in radiation dose also reduced the spectral SNR. At some dose level the duty cycle reaches a limit and cannot further be changed. If this happens, the protocol may generally use lower tube currents to further reduce the dose. This, however, is also limited. A lower tube currents will increase the transition time, in particular from high voltage plateaus to lower voltage plateaus. This increase in transition time is due to the falling transition time being mainly defined by the output capacitance of the generator and the emission current that discharges this capacitor. Hence, a low emission current will lead to a longer transition time. Increasing the transition times may significantly decrease the spectral performance, since the relative time the voltage is actually at the plateaus will be smaller and a large amount of time, the voltage will be in transition leading to a wors spectral separation of the measurement intervals. This is shown in Fig. 6, where the ramping down times are shown to be quite long, taking place during a significantly large part of the measurement intervals. At some point, the transition between the voltage plateaus may become so long that the available time for the acquisition is not long enough anymore. In the example, shown in Fig. 3, this point is reached at about 18% radiation dose. The performance graph stops below this point. Below this point, this system cannot execute a scan with the requested dose level without applying a filter 6. The effect of applying a corresponding radiation filter 6 is shown via the dashed line in Fig. 3. The dashed line shows the spectral imaging performance SNR of the system with an applied radiation filter 6. The radiation filter 6 reduces the tube output flux before radiating the object or subject, such leading to a generally lower radiation dose. This can be achieved without reducing the tube emission current, since the radiation filter 6 is applied after the generation of the x-rays. Hence, the transition time can still be relatively short. Compared to the curve corresponding to the case without filter 6 (solid line), the curve (dashed line) is shifted towards lower radiation dose values. The performance shape is similar with an optimum in the middle part of the curve and reduced performance for lower and higher dose levels. The maximum SNR is even increased compared to the curve without filter 6 due to the improved spectral separation that can be achieved with the radiation filter 6. Furthermore, lower radiation dose levels (i.e. lower than about 18%) are now within reach. The system can now perform low dose imaging. Based on the requested dose level, the CT scanner can decide on the optimal scan mode with or without filter 6. The scan may be executed with the optimal setting. For example, it may also be possible to increase the emission current while still having a relatively low radiation dose due to the applied radiation filter 6. Hence, an algorithm may be configured to optimize the spectral imaging performance based on multiple free or fixed variables, such as emission current, radiation dose, duty cycle, scan speed etc. In particular, the radiation dose and/or the scan speed may be fixed parameters. In particular the tube emission current and the duty cycle may be free parameters. The duty cycle may, for example be defined by the relative times of the higher voltage and the lower voltage of the kVp-S being applied. For example, a ratio of the time the higher voltage and the lower voltage may be a free parameter within the boundary of the desired scan speed. If there are multiple different radiation filters 6 available (e.g. comprising different materials and/or different thicknesses), multiple different curves for different radiation filters 6 may be possible, giving an even greater range of possible combinations of scan speed and radiation dose and also enabling better spectral performance at a wider range of radiation dose settings. The multiple different curves for different radiation filters 6 may in particular be shifted to different radiation doses and/or have performance maxima at different radiation doses.

Fig. 7 shows a schematic sectional view from the side of a computed tomography system according to an embodiment of the invention. The computed tomography system comprises a patient table 7 as well as a rotatable gantry 1 with an x-ray source 2 and an x-ray detector 3. The gantry 1 may rotate such that the x-ray source 2 and the x-ray detector 3 are rotated around the area above the patient table, where a patient may be placed. A control circuitry of the computed tomography system is configured to control a computed tomography scan performed by the computed tomography system. The control circuitry may be part of a control station 4. A user input functionality obtains user input settings concerning a kilovoltage peak switching computed tomography scan comprising, for example from a built-in user interface 5 or from a another device which is not part of the claimed system. The settings comprise a desired radiation dose setting and a desired speed setting of the scan. The control circuitry is configured to automatically set scan parameters depending on the user input. The system further comprises a radiation filter 6 and the system is configured to automatically determine whether a radiation filter 6 is required to achieve the desired radiation dose setting. If required depending on the set scan parameters and the desired radiation dose setting, the system is configured to place the at least one radiation filter 6 in front of the x-ray source 2. For example, the system may be configured to perform the method as described with respect to Fig. 1. Optionally, the computed tomography system may comprise a set of radiation filters 6 and may be configured to choose a filter 6 of the set of radiation filters 6 according to a defined filter suitability criterion. The suitability criterion may comprise choosing a filter 6 that provides a maximum spectral imaging performance of the plurality of filters 6 for a set radiation dose setting.

The above-discussion is intended to be merely illustrative of the present system and should not be construed as limiting the appended claims to any particular embodiment or group of embodiments. Accordingly, the specification and drawings are to be regarded in an illustrative manner and are not intended to limit the scope of the appended claims. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "an" or "a" does not exclude a plurality. A single processor or other unit may fulfil the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS

- 1: gantry
- 2: x-ray source
- 3: x-ray detector
- 4: control station
- 5: user interface
- 6: radiation filter
- 7: patient table
- 10: tube voltage
- 11: measurement interval of the x-ray detector

## Claims

**1.** A method for performing kilovoltage peak switching computed tomography with a computed tomography system comprising an x-ray source (2), the method comprising the following steps:
(a) receiving user input for a kilovoltage peak switching computed tomography scan, the user input comprising a desired radiation dose setting and a desired speed setting of the scan;
(b) setting computed tomography scan parameters based on the received user input;
(c) automatically determining whether a radiation filter (6) is required to achieve the desired radiation dose setting and desired speed setting and, if required, placing the radiation filter (6) in front of the x-ray source (2) to achieve the desired radiation dose with the set scan parameters;
(d) performing a kilovoltage peak switching computed tomography scan with the set scan parameters.

**2.** The method according to claim 1,
wherein the radiation filter (6) is adapted to improve a spectral imaging performance.

**3.** The method according to any one of the preceding claims,
wherein a performance relation between a spectral imaging performance and a radiation dose, the relation depending on a tube emission current of the x-ray source (2) and radiation filter (6) setting, is retrieved and/or determined,
wherein determining whether a radiation filter (6) is required is further based on the performance relation.

**4.** The method according to claim 3,
wherein the performance relation further depends on a duty cycle for time periods of the low and high tube voltage intervals of the kilovoltage peak switching.

**5.** The method according to any one of claims 3 to 4,
wherein the performance relation is based on a signal to noise ratio in relation to radiation dose.

**6.** The method according to any one of claims 3 to 5,
wherein it is determined that the radiation filter (6) is required if placing the filter (6) increases the expected spectral imaging performance for the desired radiation dose setting.

**7.** The method according to any one of claims 3 to 6,
wherein, in case that a radiation filter (6) is determined to be required, a radiation filter (6) out of a plurality of radiation filters is chosen that provides the maximum spectral imaging performance of the plurality of radiation filters for the desired radiation dose setting.

**8.** The method according to any one of the preceding claims,
wherein a minimum tube emission current of the x-ray source (2) that is required for kilovoltage peak switching computed tomography at the desired speed setting of the scan is retrieved and/or determined,
wherein it is determined that the radiation filter (6) is required if the desired radiation dose setting cannot be achieved above the minimum tube emission current without the radiation filter (6).

**9.** The method according to any one of the preceding claims,
wherein the radiation filter (6) has a K-edge of at least 20 keV, preferably at least 35 keV, more preferably at least 45 keV.

**11.** A computer program comprising instructions which, when the program is executed by a control circuitry of a computed tomography system, cause the computed tomography system to carry out the method according to any one of the preceding claims.

**12.** A computed tomography system, the system comprising
- a gantry (1) with an x-ray source (2);
- a control circuitry configured to control a computed tomography scan performed by the computed tomography system;
- a user input functionality for obtaining user input to choose settings concerning a kilovoltage peak switching computed tomography scan comprising a desired radiation dose setting and a desired speed setting of the scan, wherein the control circuitry is configured to automatically set scan parameters depending on the user input;
- at least one radiation filter (6), wherein the computed tomography system is configured to automatically determine whether a radiation filter (6) is required to achieve the desired radiation dose setting and, if required, place the at least one radiation filter (6) in front of the x-ray source (2) depending on the set scan parameters and the desired radiation dose setting;
wherein the computed tomography system is optionally configured to perform the method according to any one of claims 1 to 10.

**13.** The computed tomography system according to claim 12,
wherein the computed tomography system comprises a set of radiation filters (6) and is configured to, if a radiation filter (6) is determined to be required, chose a radiation filter (6) of the set of radiation filters (6) according to a defined filter suitability criterion,
wherein the suitability criterion comprises in particular choosing a radiation filter (6) that provides a maximum spectral imaging performance of the plurality of radiation filters (6) for a set radiation dose setting.

**14.** The computed tomography system according to claim 13,
wherein the radiation filter (6) or the radiation filters (6) of the set of radiation filters (6) has/have a K-edge of at least 20 keV, preferably at least 35 keV, more preferably at least 45 keV.

**15.** A use of a radiation filter (6) for lowering the radiation dose during a kilovoltage peak switching computed tomography scan depending on a desired radiation dose setting and a desired scan speed setting.
